# EUROPÄISCHE PATENTSCHRIFT

(11) **EP 0 298 210 B1**
(45) Veröffentlichungstag und Bekanntmachung des Hinweises auf die Patenterteilung: **08.12.1993**
(21) Anmeldenummer: 88106747.4
(22) Anmeldetag: 27.04.1988
(51) Int. Cl.: G01N 33/531, C07K 7/08

(54) **Verfahren zur selektiven immunologischen Bestimmung von intaktem Prokollagen Peptid (Typ III) und Prokollagen (Typ III) in Körperflüssigkeiten und Mittel zu dessen Durchführung**
Method for the selective immunological determination of intact procollagen peptide (type III) and procollagen (type III) in body fluids and reagents used therefor
Procédé pour la détermination immunologique sélective de procollagène-peptide intact (type III) et procollagène (type III) dans les fluides corporels et réactifs utilisés à cet effet

(30) Priorität: 02.05.1987 DE 3714634
(43) Veröffentlichungstag der Anmeldung: 11.01.1989
(73) Patentinhaber: HOECHST AKTIENGESELLSCHAFT, 65926 Frankfurt am Main (DE); Max-Planck-Gesellschaft zur Förderung der Wissenschaften e.V., D-37073 Göttingen (DE)
(72) Erfinder: Brocks, Dietrich, Dr., D-6200 Wiesbaden (DE); Timpl, Rupert, Dr., D-8035 Gauting (DE)

(56) Entgegenhaltungen:
- EP-A- 0 004 940
- EP-A- 0 089 008
- CLINICAL CHEMISTRY, Band 31, Nr. 8, 1985; O. NIEMELÄ, Seiten 1301-1304#
- CHEMICAL ABSTRACTS, Band 97, Nr. 1, 5. Juli 1982, Columbus, OH (US); N. SUNDARRAJ et al., Seite 444, Nr. 4431s#
- BIOLOGICAL ABSTRACTS, Band 83, Nr. 6, 1987, Philadelphia, PA (US); H. KONDO, Seite AB-267, Nr. 52561#

## Beschreibung

Prokollagen Peptid (Typ III) ist das aminoterminale Propeptid des Kollagen (Typ III), das nach Sezernierung des Prokollagen (Typ III)-Moleküls extrazellulär abgespalten wird. Mit einer radioimmunologischen Bestimmungsmethode, wie sie in der Europäischen Patentschrift Nr. 4940 beschrieben ist, kann die Konzentration dieses Prokollagen Peptids in Körperflüssigkeiten bestimmt werden. Die Kenntnis der Serumkonzentration des Peptids erlaubt Aussagen über die Aktivität fibrotischer Erkrankungen, wie z.B. der Leber [Rohde, H. et al. Eur. J. Clin. Invest 9, 451 - 459 (1979)].

Die exakte, selektive Bestimmung von Prokollagen Peptid (Typ III) in Serum und anderen Körperflüssigkeiten ist mit den bisher beschriebenen Methoden aber nicht möglich, da die polyklonalen Antikörper, die in diesen Methoden eingesetzt werden, mit verschiedenen, im Serum vorkommenden Antigenen, die zum Teil Abbauprodukte des Prokollagen Peptids (Typ III) darstellen, mit unterschiedlicher, niedrigerer Affinität reagieren [Niemelä, O. et al. Clin. Chim. Acta 124, 39 - 44 (1982)]. Dies führt dazu, daß die Serumverdünnungskurven und die Verdünnungskurven anderer Körperflüssigkeiten zur Eichkurve, die mit reinem Prokollagen Peptid (Typ III) erstellt wird, nicht parallel sind und daher von jeder unbekannten Probe der Antigengehalt in meheren Verdünnungen bestimmt werden muß, um die Antigenkonzentration über den 50 % Interzept der Verdünnungskurve zu ermitteln.

Ein weiterer Nachteil dieser Verfahren ist, daß aufgrund einer zu geringen Kreuzreaktivität der Antikörper mit Ratten- bzw. Mäuseantigen eine Bestimmung der Antigenkonzentration in Körperflüssigkeiten dieser Spezies nicht möglich war.

Mit Hilfe des Verfahrens, das von Schuppan et al. [J. Hepatol. 3,27-37 (1986)] beschrieben wurde, kann die Konzentration der Antigene im Rattenserum bestimmt werden. Allerdings hat dieses Verfahren den gleichen auch bei der Methode für Humanseren beschriebenen Nachteil [Niemelä et al. Clin. Chim. Acta 124, 39 - 44 (1982)] der nicht parallelen Inhibitionskurven bei verschiedenen Körperflüssigkeiten.

Mit Hilfe des Verfahrens der Europäischen Patentanmeldung 0089008 ist es möglich, dieses technische Problem zu lösen, indem Antikörper eingesetzt werden, die für intaktes Prokollagen Peptid (Typ III) und sein Abbauprodukt Col 1 vergleichbare Affinität haben. Mit diesem Verfahren werden intaktes und degradiertes Prokollagen Peptid (Typ III) gemeinsam bestimmt, was jedoch zur Ungenauigkeit in der diagnostischen Aussage führt, da Normalkollektiv und Patientenkollektiv stark überlappen können.

Überraschenderweise wurde nun gefunden, daß bei Verwendung eines Peptids mit einer bestimmten Aminosäuresequenz für die Immunisierung Antikörper erhalten werden können, mit Hilfe derer eine spezifische Bestimmung des intakten Prokollagen Peptids (Typ III) möglich ist. Mit diesen Antikörpern kann überraschenderweise auch der Prokollagen Peptid (Typ III)-Gehalt in Körperflüssigkeiten von Ratte oder Maus bestimmt werden, was für die tierexperimentelle Erprobung fibrosuppressiver Substanzen von Nutzen ist.

Die Erfindung betrifft somit ein Verfahren zur immunologischen Bestimmung von aminoterminalem Prokollagen Peptid (Typ III) mit Antikörpern, das dadurch gekennzeichnet ist, daß
a) Tiere mit einem Peptid der Sequenz
   I-C-E-S-C-P-T-G-G-Q-N-Y-S-P,
   gebunden an ein immunogenes Protein immunisiert werden,
b) aus dem Serum die Antikörper gewonnen werden, die mit intaktem aminoterminalen Prokollagen Peptid (Typ III) reagieren und
c) die Menge des aminoterminalen Prokollagen Peptids (Typ III) und des Prokollagens (Typ III) über den gebildeten Antigen-Antikörper-Komplex bestimmt wird.

Ferner betrifft die Erfindung Antikörper, die durch Immunisierung von Tieren mit obengenanntem Peptid entstehen, das an ein immunogenes Protein gebunden ist.

Im folgenden wird die Erfindung detailliert beschrieben, insbesondere in ihren bevorzugten Ausführungsformen. Ferner wird die Erfindung in den Ansprüchen definiert.

Zur Herstellung der Antikörper werden Tiere, bevorzugt Nagetiere, wie z.B. Kaninchen, Meerschweinchen, oder Ziege und Schaf mit dem Peptid der Sequenz
I-C-E-S-C-P-T-G-G-Q-N-Y-S-P
gekoppelt an ein geeignetes immunogenes Protein in Gegenwart von komplettem Adjuvans immunisiert. Besonders bevorzugt werden Kaninchen eingesetzt. Mit wiederholten Sekundärinjektionen, beispielsweise im Abstand von 4 bis 8 Wochen, wird die Immunantwort verstärt. Der Erfolg der Immunisierung wird durch Bestimmung der Konzentration von Antikörpern im radioimmunologischen Bindungstest kontrolliert [R. Timpl und L. Risteli, Immunochemistry of the extracellular matrix, H. Furthmayr Ed., Vol. 1, 199 (1982)].

Geeignete Proteine, an die das genannte Peptid gebunden sein kann sind alle immunogenen Proteine. Bevorzugt werden Hämocyanin, Albumin oder Polylysin verwendet. Das Peptid kann nach dem Fachmann bekannten Methoden hergestellt werden, wie z.B. von G. Barany und A. B. Merrifield in The Peptides Vol. 2, pp 3-254 (1980), Academic Press oder E. Brown, R. C. Sheppard und B. J. Williams, J. Chem. Soc. Perkin Transact. 1, 1161 (1983) beschrieben.

Die erfindungsgemäßen Antikörper können als Serum oder gereinigt in verschiedenen immunologischen Verfahren, einschließlich allen Formen des Radioimmunassays, z.B. sequentielle Sättigungsanalyse oder Gleichgewichtsanalyse, gegebenenfalls nach Markierung mit Chloramin T oder Bolton-Hunter-Reagenz [Felber, Meth. Biochem. Anal. 22, 1 (1974); Shelley et All, Clin. Chem. 19, 146 (1975)] sowie in anderen kompetitiven Bindungsassays, wie Fluoreszens-, Enzymimmunoassay, Chemiluminestenz oder anderen Immunoassays verwendet werden. Die Antikörper können daher in immunologischen Verfahren zur Isolierung und Charakterisierung sowie zur quantitativen Bestimmung von Prokollagen Peptid (Typ III) in Geweben und Körperflüssigkeiten eingesetzt werden. Zur quantitativen Bestimmung verfährt man nach den dem Fachmann an sich bekannten Methoden, indem eine flüssige Probe, die Prokollagen Peptid (Typ III) enthält, mit den erfindungsgemäßen Antikörpern zur Reaktion gebracht wird und die Menge des Prokollagen Peptids (Typ III) über den gebildeten Antigen-Antikörper-Komplex bestimmt wird. Dabei spielt es keine Rolle, ob das Prokollagen Peptid (Typ III) noch mit dem Aminoterminus des Prokollagen (Typ III) verknüpft ist oder nicht. Die bislang bei der immunologischen Bestimmung störenden Degradationsprodukte des Prokollagen Peptids (Typ III), insbesondere das Col 1, werden von den erfindungsgemäßen Antikörpern nicht miterfaßt.

In den folgenden Beispielen wird die Erfindung weitergehend erläutert. Prozentangaben beziehen sich auf das Gewicht, wenn nicht anders angegeben.

### Beispiel 1

### Herstellung der Peptid-Hämocyanin Konjugat-Verbindung

100 mg Hämocyanin (dialysiert gegen 5 x 3 l Wasser und dann lyophilisiert) löst man in 3 ml Wasser und gibt 24 mg des Peptids I-C-E-S-C-P-T-G-G-Q-N-Y-S-P zu. Unter Rühren bei Raumtemperatur setzt man im Verlauf von 2 Stunden in Portionen insgesamt 1 g N-Cyclohexyl-N′-[2-(4-morpholinyl)-ethyl]carbodiimid-methyl-p-toluolsulfonat zu. Nach Rühren über Nacht dialysiert man das Reaktionsgemisch 24 Stunden gegen insgesamt 5 x 10 l Wasser, und isoliert das Produkt anschließend durch Gefriertrocknung. Man erhält 137 mg der Konjugatverbindung.

### Beispiel 2

### Radioimmunbindungstest

300 µl Anitserum vom Kaninchen, immunisiert mit dem gemäß Beispiel 1 hergestellten Konjugat, in geeigneter Verdünnung werden mit 100 µl einer Prokollagen Peptid Typ III Lösung (1 ng Protein/100 µl, hergestellt wie in EP 4940, Beispiel 1, beschrieben), welches mit ¹²⁵J radioaktiv markiert ist, über Nacht inkubiert. Die gebildeten Antigen-Antikörper-Komplexe werden durch Zugabe eines gegen Immunglobulin G der zur Immunisierung verwendeten Spezies gerichteten Antiserums einer anderen Spezies ausgefällt. Nach Zentrifugation und Dekantieren des Überstandes wird die Menge der präzipitierten Radioaktivität im γ-Szintillationsspektrometer bestimmt.

### Beispiel 3

### Radioimmun-Test

0,2 ml der zu analysierenden Probe oder des Prokollagen Peptid (Typ III)-Standards werden mit einer in Bezug auf die Menge des markierten Antigens limitierenden Menge von Antiserum (in 0,1 ml Puffer) über Nacht bei 4°C inkubiert. Nach Zugabe von 0,1 ml ¹²⁵J markiertem Prokollagen Peptid (Typ III) (enthält 1 ng Protein) wird 6 - 8 Stunden bei 4°C inkubiert. Die gebildeten Antigen-Antikörper-Komplexe werden mittels eines gegen IgG der zur Immunisierung verwendeten Spezies gerichteten Antiserums präzipitiert. Nach Abzentrifugation und Dekantierung des Überstandes wird die gefällte Radioaktivität im γ-Szintillationsspektrometer bestimmt.

Durch Vergleich mit einer Eichkurve, zu deren Erstellung Standards mit unterschiedlichen Konzentrationen von Prokollagen Peptid (Typ III) eingesetzt werden, kann dann die Konzentration von Prokollagen Peptid (Typ III) in der unbekannten Lösung bestimmt werden.

### Beispiel 4

### Bestimmung der Molekulargewichtsverteilung der mit dem erfindungsgemäßen Antikörpern reagierenden Antigene im Serum von z.B. Rind und vom Schwein

1 ml Serum werden per Gelfiltrationschromatographie über eine Sephacryl S 300® Säule (1,6 x 130 cm), äquilibriert in "phosphate buffered saline" PBS mit 0,04 % Tween® 20, aufgetrennt. Je 0.2 ml der Fraktionen (jede 2,8 ml) werden in den Radioimmun-Test nach Beispiel 3 eingesetzt.

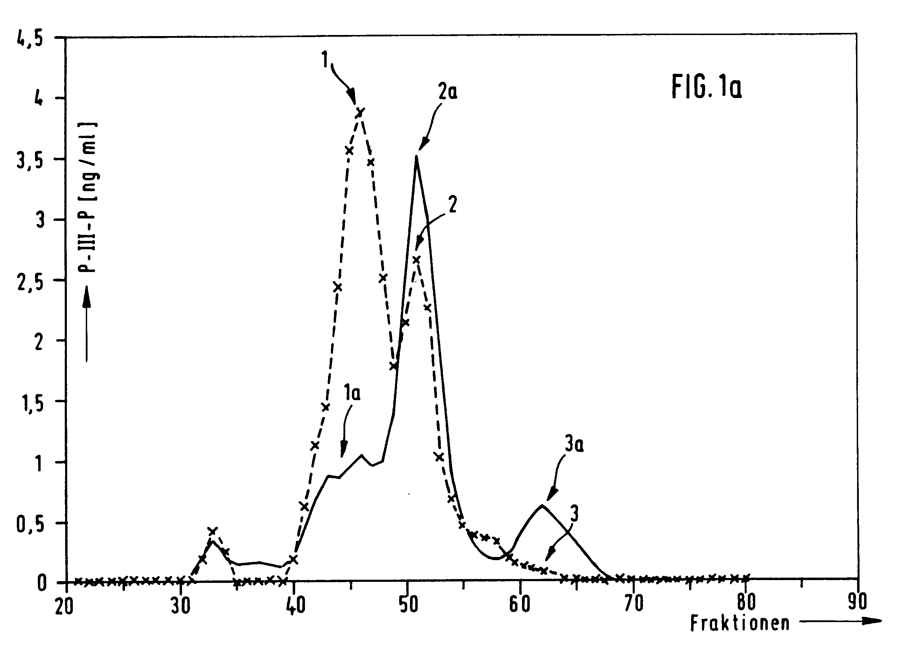

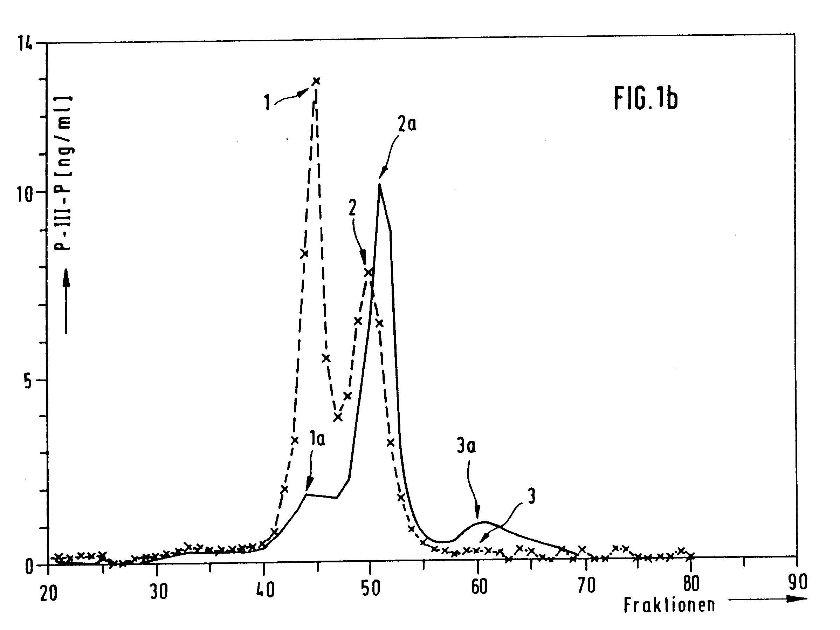

Abb 1a zeigt das Elutionsprofil des Antigens aus Schweineserum, Abb. 1b das des Antigens aus Rinderserum im Vergleich zum Profil des nach EP 4940 bestimmten Antigens. Die Peaks 1/1a entsprechen intaktem Prokollagen Typ III bzw. pN-Kollagen Typ III (Prokollagen, dem das C-terminale Ende fehlt); Peaks 2/2a entsprechen intaktem aminoterminalem Prokollagen Peptid Typ III; Peaks 3/3a entsprechen Col 1 sowie Abbauprodukten des aminoterminalen Prokollagen Peptids Typ III mit gleichem Molekulargewicht wie Col 1.

## Patentansprüche (Patentansprüche für folgende(n) Vertragsstaat(en): AT, BE, CH, DE, FR, GB, IT, LI, LU, NL, SE)

1. Verfahren zur immunologischen Bestimmung von aminoterminalem Prokollagen Peptid (Typ III) mit Antikörpern, dadurch gekennzeichnet, daß
a) Tiere mit einem Peptid der Sequenz
I-C-E-S-C-P-T-G-G-Q-N-Y-S-P
gebunden an ein immunogenes Protein immunisiert werden,
b) aus dem Serum die Antikörper gewonnen werden, die mit intaktem aminoterminalem Prokollagen Peptid (Typ III) reagieren und
c) die Menge des aminoterminalen Prokollagen Peptids (Typ III) und/oder des Prokollagen Typ III über den gebildeten Antigen-Antikörper-Komplex bestimmt wird.

2. Verfahren nach Anspruch 1, dadurch gekennzeichnet, daß das Peptid an Hämocyanin, Albumin oder Polylysin gebunden ist.

3. Verfahren nach Anspruch 1 oder 2, dadurch gekennzeichnet, daß Nagetiere oder Ziege oder Schaf immunisiert werden.

4. Verfahren nach Anspruch 3, dadurch gekennzeichnet, daß Kaninchen immunisiert werden.

5. Antikörper erhältlich durch Immunisierung von Tieren mit dem Peptid der Sequenz
I-C-E-S-C-P-T-G-G-Q-N-Y-S-P
gebunden an ein immunogenes Protein.

6. Antikörper nach Anspruch 5, dadurch gekennzeichnet, daß das Peptid an Hämocyanin, Albumin oder Polylysin gebunden ist.

7. Antikörper nach Anspruch 5 oder 6, dadurch gekennzeichnet, daß Nagetiere oder Ziege oder Schaf immunisiert werden.

8. Antikörper nach Anspruch 7, dadurch gekennzeichnet, daß Kaninchen immunisiert werden.

9. Verbindung bestehend aus dem Peptid der Sequenz
I-C-E-S-C-P-T-G-G-Q-N-Y-S-P
gebunden an ein immunogenes Protein.

10. Verwendung der Verbindung nach Anspruch 9 als Antigen.

## Patentansprüche (Patentansprüche für folgende(n) Vertragsstaat(en): ES, GR)

1. Verfahren zur immunologischen Bestimmung von aminoterminalem Prokollagen Peptid (Typ III) mit Antikörpern, dadurch gekennzeichnet, daß
a) Tiere mit einem Peptid der Sequenz
I-C-E-S-C-P-T-G-G-Q-N-Y-S-P
gebunden an ein immunogenes Protein immunisiert werden.
b) aus dem Serum die Antikörper gewonnen werden, die mit intaktem aminoterminalem Prokollagen Peptid (Typ III) reagieren und
c) die Menge des aminoterminalen Prokollagen Peptids (Typ III und/oder des Prokollagen Type III über den gebildeten Antigen-Antikörper-Komplex bestimmt wird.

2. Verfahren nach Anspruch 1, dadurch gekennzeichnet, daß das Peptid an Hämocyanin, Albumin oder Polylysin gebunden ist.

3. Verfahren nach Anspruch 1 oder 2, dadurch gekennzeichnet, daß Nagetiere oder Ziege oder Schaf immunisiert werden.

4. Verfahren nach Anspruch 3, dadurch gekennzeichnet, daß Kaninchen immunisiert werden.

## Claims (Claims for the following Contracting State(s): AT, BE, CH, DE FR, GB, IT, LI, LU, NL, SE)

1. A method for the immunological determination of amino-terminal procollagen peptide (type III) using antibodies, which comprises
a) immunization of animals with a peptide of the sequence
I-C-E-S-C-P-T-G-G-Q-N-Y-S-P
bound to an immunogenic protein,
b) obtaining from the serum the antibodies which react with intact amino-terminal procollagen peptide (type III) and
c) determination of the amount of amino-terminal procollagen peptide (type III) and/or of procollagen type III via the antigen-antibody complex which is formed.

2. The method as claimed in claim 1, wherein the peptide is bound to hemocyanin, albumin or polylysine.

3. The method as claimed in claim 1 or 2, wherein rodents or goats or sheep are immunized.

4. The method as claimed in claim 3, wherein rabbits are immunized.

5. Antibodies obtainable by immunization of animals with the peptide of the sequence
I-C-E-S-C-P-T-G-G-Q-N-Y-S-P
bound to an immunogenic protein.

6. Antibodies as claimed in claim 5, wherein the peptide is bound to hemocyanin, albumin or polylysine.

7. Antibodies as claimed in claim 5 or 6, wherein rodents or goats or sheep are immunized.

8. Antibodies as claimed in claim 7, wherein rabbits are immunized.

9. A compound composed of the peptide of the sequence
I-C-E-S-C-P-T-G-G-Q-N-Y-S-P
bound to an immunogenic protein.

10. The use of the compound as claimed in claim 9 as an antigen.

## Claims (Claims for the following Contracting State(s): ES, GR)

1. A method for the immunological determination of amino-terminal procollagen peptide (type III) using antibodies, which comprises
a) immunization of animals with a peptide of the sequence
I-C-E-S-C-P-T-G-G-Q-N-Y-S-P
bound to an immunogenic protein,
b) obtaining from the serum the antibodies which react with intact amino-terminal procollagen peptide (type III) and
c) determination of the amount of amino-terminal procollagen peptide (type III) and/or of procollagen type III via the antigen-antibody complex which is formed.

2. The method as claimed in claim 1, wherein the peptide is bound to hemocyanin, albumin or polylysine.

3. The method as claimed in claim 1 or 2, wherein rodents or goats or sheep are immunized.

4. The method as claimed in claim 3, wherein rabbits are immunized.

## Revendications (Revendications pour l'(les) Etat(s) contractant(s) suivant(s): AT, BE, CH, DE, FR, GB, IT, LI, LU, NL, SE)

1. Procédé pour la détermination immunologique de peptide aminoterminal du procollagène (type III) à l'aide d'anticorps, caractérisé en ce que :
a) on immunise des animaux avec un peptide de séquence
I-C-E-S-C-P-T-G-G-Q-N-Y-S-P
lié à une protéine immunogène,
b) à partir du sérum, on récupère les anticorps qui réagissent avec le peptide aminoterminal intact du procollagène (type III), et
c) on détermine la quantité du peptide aminoterminal du procollagène (type III) et/ou la quantité de procollagène de type III d'après le complexe antigène-anticorps formé.

2. Procédé selon la revendication 1, caractérisé en ce que le peptide est lié à l'hémocyanine, à l'albumine ou à la polylysine.

3. Procédé selon la revendication 1 ou 2, caractérisé en ce qu'on immunise des rongeurs ou des chèvres ou des moutons.

4. Procédé selon la revendication 3, caractérisé en ce qu'on immunise des lapins.

5. Anticorps qu'on peut obtenir par immunisation d'animaux avec le peptide de séquence
I-C-E-S-C-P-T-G-G-Q-N-Y-S-P
lié à une protéine immunogène.

6. Anticorps selon la revendication 5, caractérisé en ce que le peptide est lié à l'hémocyanine, à l'albumine ou à la polylysine.

7. Anticorps selon la revendication 5 ou 6, caractérisé en ce qu'on immunise des rongeurs ou des chèvres ou des moutons.

8. Anticorps selon la revendication 7, caractérisé en ce qu'on immunise des lapins.

9. Composé constitué du peptide de séquence
I-C-E-S-C-P-T-G-G-Q-N-Y-S-P
lié à une protéine immunogène.

10. Utilisation du composé selon la revendication 9 comme antigène.

## Revendications (Revendications pour l'(les) Etat(s) contractant(s) suivant(s): ES, GR)

1. Procédé pour la détermination immunologique de peptide aminoterminal du procollagène (type III) à l'aide d'anticorps, caractérisé en ce que :
a) on immunise des animaux avec un peptide de séquence
I-C-E-S-C-P-T-G-G-Q-N-Y-S-P
lié à une protéine immunogène,
b) à partir du sérum, on récupère les anticorps qui réagissent avec le peptide aminoterminal intact du procollagène (type III), et
c) on détermine la quantité du peptide aminoterminal du procollagène (type III) et/ou la quantité de procollagène de type III, d'après le complexe antigène-anticorps formé.

2. Procédé selon la revendication 1, caractérisé en ce que le peptide est lié à l'hémocyanine, à l'albumine ou à la polylysine.

3. Procédé selon la revendication 1 ou 2, caractérisé en ce qu'on immunise des rongeurs ou des chèvres ou des moutons.

4. Procédé selon la revendication 3, caractérisé en ce qu'on immunise des lapins.
